(19) **Europäisches Patentamt**
**European Patent Office**
**Office européen des brevets**

(11) **EP 4 571 344 A1**

(12) **EUROPÄISCHE PATENTANMELDUNG**

(43) Veröffentlichungstag:
**18.06.2025  Patentblatt 2025/25**

(21) Anmeldenummer: **23216088.7**

(22) Anmeldetag: **13.12.2023**

(51) Internationale Patentklassifikation (IPC):
*G01R 33/565* *(2006.01)*

(52) Gemeinsame Patentklassifikation (CPC):
**G01R 33/56509**

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(71) Anmelder: **Siemens Healthineers AG
91301 Forchheim (DE)**

(72) Erfinder:
• **Sukkau, Johann
91074 Herzogenaurach (DE)**
• **Wohlers, Julian
91052 Erlangen (DE)**

(74) Vertreter: **Siemens Healthineers
Patent Attorneys
Postfach 22 16 34
80506 München (DE)**

(54) **BEWEGUNGSVERFOLGUNG EINES OBJEKTBEREICHS IN EINEM UNTERSUCHUNGSTUNNEL EINES MAGNETRESONANZTOMOGRAPHIESYSTEMS**

(57)    Verfahren zur Bewegungsverfolgung eines Objektbereichs in einem Untersuchungstunnel (6) eines Magnetresonanztomographiesystems, wobei das Verfahren die folgenden Schritte umfasst: Anordnen einer Trägereinheit (1) um den Objektbereich herum, sodass die Position der Trägereinheit (1) mit der Position des Objektbereichs verknüpft ist, wobei die Trägereinheit (1) insbesondere eine Magnetresonanztomographie-Körperspule ist, wobei an der Trägereinheit (1) zumindest ein Bewegungssensor angeordnet ist; Festlegen einer Ausgangsposition des Objektbereichs; Überwachen der von dem zumindest einen Bewegungssensor erfassten Bewegung, um Bewegungsdaten zu erhalten; Bestimmen einer aktuellen Position des Objektbereichs relativ zu der Ausgangsposition basierend auf den mit dem zumindest einen Bewegungssensor erfassten Bewegungsdaten.

FIG 7

**Beschreibung**

Die Erfindung betrifft ein Verfahren zur Bewegungsverfolgung eines Objektbereichs in einem Untersuchungstunnel eines Magnetresonanztomographiesystems, eine Messvorrichtung und ein Magnetresonanztomographiesystem.

Unabhängig vom grammatikalischen Geschlecht eines bestimmten Begriffs sind Personen mit männlicher, weiblicher oder anderer Geschlechteridentität mit umfasst.

Magnetresonanztomographie basiert typischerweise auf der Aufnahme von Schichtbildern. Eine Problematik ist dabei die Ausrichtung der jeweiligen Schicht relativ zu einem zu untersuchenden Objektbereich. Im Stand der Technik gibt es bereits seit einigen Jahren Verfahren zum automatischen Selektieren von Elementen im Sichtfeld (englisch "Field of View" - kurz FOV), die auf Magnetresonanzmessungen und beispielsweise festgelegten Orientierungspunkten ("Landmarks") basieren. Im Englischen ist für solche Verfahren der Begriff "auto align" (zu Deutsch etwa "automatisches Ausrichten) üblich. Es kann jedoch immer wieder zu Fehlern bei dem automatischen Selektieren kommen, die beispielsweise mit ungewöhnlichen Anatomien oder Bewegung des zu untersuchenden Bereichs begründet sind. Insbesondere bei der Bildgebung von Knie und Ellbogen kann es recht häufig zu einer Bewegung und Rotation des Objektbereichs kommen. Bewegung des Objektbereichs zwischen einzelnen Scanschritten kann problematisch sein, weil typische Verfahren zum Ausrichten auf einem ersten Lokalizer-Bild basieren, welches bei Bewegung des Objektsbereichs die aktuelle Position im Verlauf des Scans zunehmend schlecht charakterisiert. Dementsprechend erhöht sich typischerweise das Risiko eines fehlerhaft eingestellten FOV bei späteren Scanschritten bzw. gegen Ende des Scans.

Das Problem wird teilweise dadurch angegangen, dass ein Radiologe die Position und Orientierung der jeweiligen Schichten gegebenenfalls manuell korrigiert. Diese Lösung ist jedoch zeitaufwendig und oft mühsam für den Radiologen.

Für Messungen im Bereich der Magnetresonanztomographie ist es vorteilhaft, wenn Empfangsspulen möglichst nahe an einer zu messenden Stelle sind, um ein gutes Signal-Rausch-Verhältnis (auch SNR von englisch Signal-to-Noise Ratio) zu erzielen. Daher werden für den Empfang von Signalen Lokalspulen oftmals direkt an dem zu untersuchenden Objektbereich platziert. Es ist jedoch nicht einfach die Position der Lokalspule genau zu bestimmen. Es gibt im Stand der Technik (siehe DE 10 2016 203 255 A1 bzw. US 2017 / 0248665 A1) den Ansatz, eine Position einer Lokalspule mit Hilfe eines Magnetfeldsensors zu bestimmen, wobei Kenntnisse über den Magnetfeldverlauf um einen Untersuchungstunnel des Magnetresonanztomographiesystems als Grundlage dienen. Das Magnetfeld im Untersuchungstunnel selbst ist jedoch in der Regel homogen, womit diese Positionsbestimmung nicht direkt während einer Messung angewendet werden kann.

Die Veröffentlichung EP 4 170 375 A1 betrifft eine Positionsbestimmung einer Spule im Untersuchungstunnel eines Magnetresonanztomographiesystems. Dabei kann eine Position der Spule mit Hilfe eines Markerelements und einer Magnetresonanztomographiemessung bestimmt werden. Für die eigentliche Messung an einem Objekt wird das Markerelement deaktiviert. Damit ist eine Positionsbestimmung in einem Untersuchungstunnel möglich. Indem das Markerelement jedoch während der eigentlichen Messung deaktiviert wird, kann eine Positionsverfolgung während der eigentlichen Messung typischerweise ebenfalls nicht erfolgen.

Es ist daher eine Aufgabe der vorliegenden Erfindung, eine Möglichkeit bereitzustellen, mit der die oben genannten Probleme zumindest teilweise gelöst werden können. Insbesondere soll eine Möglichkeit zur Bewegungsverfolgung eines Objektbereichs in einem Untersuchungstunnel eines Magnetresonanztomographiesystems, vorzugsweise auch während einer Messung, gefunden werden.

Diese Aufgabe wird gelöst durch ein Verfahren gemäß Anspruch 1, eine Messvorrichtung gemäß Anspruch 11 und ein Magnetresonanztomographiesystem gemäß Anspruch 15. Weitere Merkmale und Vorteile ergeben sich aus den abhängigen Ansprüchen, der Beschreibung und den beigefügten Figuren.

Gemäß einem ersten Aspekt der Erfindung ist ein Verfahren zur Bewegungsverfolgung eines Objektbereichs in einem Untersuchungstunnel eines Magnetresonanztomographiesystems vorgesehen. Das Verfahren umfasst die folgenden Schritte:

(a) Anordnen einer Trägereinheit um den Objektbereich herum, sodass die Position der Trägereinheit mit der Position des Objektbereichs verknüpft ist, wobei die Trägereinheit insbesondere eine Magnetresonanztomographie-Körperspule ist, wobei an der Trägereinheit zumindest ein Bewegungssensor angeordnet ist;
(b) Festlegen einer Ausgangsposition des Objektbereichs;
(c) Überwachen der von dem zumindest einen Bewegungssensor erfassten Bewegung, um Bewegungsdaten zu erhalten;
(d) Bestimmen einer aktuellen Position des Objektbereichs relativ zu der Ausgangsposition basierend auf den mit dem zumindest einen Bewegungssensor erfassten Bewegungsdaten.

Der Begriff Objektbereich ist im Sinne der Erfindung breit zu verstehen. Generell kann der Objektbereich irgendein Bereich eines Objekts sein. Der Objektbereich kann beispielsweise ein Region of Interest (kurz "ROI", zu Deutsch "Bereich von Interesse") sein bzw. einem Region of Interest entsprechen. Der Objektbereich kann ein Teilbereich des Objekts oder der gesamte Bereich des Objekts sein. Der Objektbereich kann insbesondere ein Bereich sein, der im Rahmen einer Magnetresonanztomographie(MRT)-Untersuchung untersucht werden soll. Das Objekt kann beispielsweise ein Teil eines Lebewesens, insbesondere eines Tiers oder eines Menschen sein. Beispiel kann das Objekt Teil eines Patienten sein. Das Objekt kann eine anatomische Struktur und/oder ein Organ sein. Beispielsweise kann das Objekt ein Gelenk, wie ein Knie oder Ellbogen sein. Gerade ein Gelenk, wie ein Knie oder ein Ellbogen, kann im Stand der Technik aufgrund der Bewegungsfreiheitsgrade zu Schwierigkeiten bei einem automatischen Selektieren dieses Bereichs führen. Vorteilhafterweise kann es mit dem erfindungsgemäßen Verfahren zur Bewegungsverfolgung des Objektbereichs möglich sein, den Objektbereich selbst während einer Messung in dem an sich nur eingeschränkt für eine Bewegungsverfolgung zugänglichen Untersuchungstunnel eines MRT-Systems zu ermöglichen.

Im Rahmen des Verfahrens wird eine Trägereinheit um den Objektbereich herum angeordnet. Die Trägereinheit kann teilweise oder vollständig um den Objektbereich herum angeordnet werden. Die Trägereinheit kann zumindest bereichsweise flexibel, beispielsweise biegsam, ausgestaltet sein. Die Trägereinheit kann beispielsweise um den Objektbereich herumgewickelt oder gefaltet werden. Die Trägereinheit kann beweglich miteinander verbundene Glieder umfassen. Die Trägereinheit kann in einer Vorzugsrichtung flexibel, insbesondere wickelbar oder faltbar, sein. Es kann vorgesehen sein, dass die Trägereinheit nur in einer Vorzugsrichtung flexibel ist. Mit einer Flexibilität nur in der Vorzugsrichtung kann beispielsweise ein fehlerhaftes Anordnen der Trägereinheit verhindert werden. Die Vorzugsrichtung bezieht sich insbesondere auf eine Rotationsrichtung bzw. auf eine Wickelrichtung. Insbesondere kann die Trägereinheit starr in zumindest einer Richtung sein, welche nicht die Vorzugsrichtung ist/sind. Die Trägereinheit wird so um den Objektbereich herum angeordnet, dass die Position der Trägereinheit mit der Position des Objektbereichs verknüpft ist. Beispielsweise kann die Trägereinheit mit dem Objektbereich mittelbar oder unmittelbar verbunden sein. Eine mittelbare Verbindung kann beispielsweise vorliegen, wenn die Trägereinheit mit einem Element verbunden ist, dass wiederum mit dem Objektbereich verbunden ist. Beispielsweise kann die Trägereinheit durch Herumwickeln mit dem Objektbereich verbunden sein bzw. positionell verknüpft sein. Vorteilhafterweise kann durch die Verknüpfung der Position der Trägereinheit mit der Position des Objektbereichs ermöglicht werden, dass über die Position der Trägereinheit die Position des Objektbereichs bestimmt werden kann. Vorzugsweise ist die Trägereinheit so mit dem Objektbereich verknüpft, dass sie mit Bezug auf den Objektbereich translationsinvariant und/oder rotationsinvariant ist. Vorzugsweise kann die Trägereinheit gemäß einer festgelegten relativen örtlichen Beziehung zu dem Objektbereich angeordnet werden. Beispielsweise kann ein definierter Ort der Trägereinheit an einer definierten Stelle relativ zu dem Objektbereich angeordnet werden. Die Trägereinheit kann eine Markierung umfassen, mit Hilfe derer die Position der Trägereinheit relativ zu dem Objektbereich festgelegt werden kann. Die Markierung kann beispielsweise ein Muster sein oder umfassen. Beispielsweise kann die Markierung ein Kreuz umfassen. Die Markierung kann beispielsweise direkt über dem Objektbereich, insbesondere über einer festgelegten Stelle des Objektbereichs angeordnet werden. Beispielsweise kann die festgelegte Stelle das Zentrum des Objektbereichs sein. Beispielsweise kann das Muster über einem Zentrum eines Ellbogens oder eines Knies angeordnet werden. Vorzugsweise wird die Trägereinheit, insbesondere die Markierung, möglichst nahe zu dem Objektbereich angeordnet. Eine Orientierung der Trägereinheit kann relativ zu der Orientierung des Objektbereichs ausgerichtet werden. Beispielsweise kann die Markierung, insbesondere ein Muster der Markierung, zu dem Objektbereich ausgerichtet werden.

Die Trägereinheit kann insbesondere eine Magnetresonanztomographie-Körperspule sein. Vorteilhafterweise kann eine Körperspule, die ohnehin nahe zu dem Objektbereich platziert werden soll, somit auch als Trägereinheit verwendet werden. Die Körperspule kann zumindest in einer Richtung flexibel ausgestaltet sein, sodass sie um den Objektbereich herumgewickelt werden kann. Alternativ kann aber auch eine Trägereinheit verwendet werden, die keine Körperspule ist. Beispielsweise kann die Trägereinheit zusätzlich zu der Körperspule um den Objektbereich herum angeordnet werden. Beispielsweise kann die Trägereinheit, die keine Körperspule ist, verwendet werden, falls keine Körperspule um den Objektbereich herum vorgesehen ist oder falls eine Körperspule vorgesehen ist.

An der Trägereinheit ist zumindest ein Bewegungssensor angeordnet. Der zumindest eine Bewegungssensor kann insbesondere ortsfest an der Trägereinheit fixiert sein. Vorteilhafterweise kann eine Bewegung des Bewegungssensor somit direkt mit der Bewegung der Trägereinheit und insbesondere mit der Bewegung des Objektbereichs zusammenhängen. Ein Bewegungssensor ist im Rahmen der Erfindung breit zu verstehen und ist generell ein Sensor, der auf irgendeine Art eine Bewegung bzw. eine mit einer Bewegung zusammenhängende Größe erfasst. Beispielsweise kann der Bewegungssensor dazu ausgestaltet sein, eine Bewegung in eine elektrische Größe umzusetzen. Die elektrische Größe kann beispielsweise ein Strom, eine Spannung oder eine Kapazität sein. Der Bewegungssensor kann beispielsweise dazu ausgestaltet sein, einen Ortswechsel, eine Geschwindigkeit und/oder eine Beschleunigung zu erfassen. Der Bewegungssensor kann beispielsweise ein Beschleunigungssensor sein. Der Bewegungssensor, insbesondere Beschleunigungssensor, kann dazu ausgestaltet sein eine Bewegung bzw. Beschleunigung in einer bestimmten Richtung zu erfassen und/oder eine Bewegung bzw. Beschleunigung mit einer Richtungsinformation zu erfassen. Es können mehrere Bewegungssensoren, insbesondere Beschleunigungssensoren, vorgesehen sein. Die Sensoren können an verschiedenen Orten der Trägereinheit vorgesehen sein. Der zumindest eine Bewegungssensor, insbesondere Beschleunigungssensor, kann ein dreidimensionaler Bewegungssensor (3D-Bewegungssensor) bzw. dreidimensionaler Beschleunigungssensor (3D-Beschleunigungsensor) sein. Ein dreidimensionaler Sensor ist im Rahmen dieser Erfindung so zu verstehen, dass er eine entsprechende Sensorik für drei Raumrichtungen aufweist. Ein 3D-Bewegungssensor kann Bewegungsdaten für drei Raumrichtungen separat aufnehmen. Beispielsweise kann eine Bewegung in x-Richtung, eine Bewegung in y-Richtung und eine Bewegung in z-Richtung jeweils gesondert erfassbar sein. Ein 3D-Beschleunigungs-sensor kann dazu ausgestaltet sein, eine Beschleunigung in jede von drei Raumrichtungen einzeln separat detektieren zu können. Vorteilhafterweise kann somit eine Bewegung bzw. Beschleunigung zusammen mit einer Richtung der Bewegung bzw. Beschleunigung erfassbar sein. Beispielsweise kann ein 3D-Beschleunigungsensor dafür verwendet werden, um eine Orientierung im Gravitationsfeld der Erde zu bestimmen. Der Beschleunigungssensor kann somit auch als Orientierungssensor verwendet werden. Zusätzlich zu dem zumindest einen Bewegungssensor können einer oder mehrere weitere Sensoren bzw. zumindest eine weitere Art von Sensoren, an der Trägereinheit vorgesehen sein. Die zumindest eine weitere Art von Sensoren kann insbesondere zumindest ein Sensor sein, der kein Bewegungssensor ist. Beispielsweise kann zumindest ein Magnetfeldsensor bzw. Hall-Sensor, an der Trägereinheit vorgesehen sein. Weitere Sensoren können beispielsweise für das Festlegen der Ausgangsposition verwendet werden.

Vorzugsweise vor dem Verfolgen der Bewegung mit dem zumindest einen Bewegungssensor wird eine Ausgangsposition des Objektbereichs festgelegt. Die Ausgangsposition kann optional eine Ausgangsorientierung umfassen. In manchen Fällen kann es auch eine Option sein, die Ausgangsposition nachträglich festzulegen. Beispielsweise kann eine Endposition bestimmt werden, und die Ausgangsposition kann anhand der erfassten Bewegung zurückgerechnet werden. Die Ausgangsposition kann von einem Sensor, z.B. einem Positionssensor, an der Trägereinheit festgelegt bzw. bestimmt werden. Es kann vorgesehen sein, die Ausgangsposition des Objektbereichs außerhalb des Untersuchungstunnels zu bestimmen. Typischerweise ist es einfacher eine Position außerhalb des Untersuchungstunnels zu bestimmen, weil dort sowohl ein optischer Zugang, z.B. für eine Kamera, einfacher ist, das Magnetfeld weniger stark ist und auch mehr Platz ist. Die Ausgangsposition kann vorzugsweise automatisch festgelegt werden. Ein automatisches Festlegen kann beispielsweise über eine Detektionseinrichtung festgelegt werden. Die Detektionseinrichtung kann eine Detektionseinheit und eine Auswerteeinheit umfassen. Die Auswerteeinheit kann beispielsweise Teil einer Kontrolleinheit des MRT-Systems sein bzw. in die Kontrolleinheit integriert sein. Die Detektionseinheit kann beispielsweise ein Sensor und/oder eine Kamera sein. Die Kamera kann beispielsweise eine optische Kamera und/oder eine Infrarotkamera sein. Der Sensor kann beispielsweise ein Magnetfeldsensor sein. Mit dem Magnetfeldsensor kann eine Position insbesondere anhand der Magnetfeldstärke außerhalb des Untersuchungstunnels abgeschätzt werden. Beispielsweise kann die Ausgangsposition bestimmt werden, wenn eine Patientenliege in einer Ausgangsposition ist (z.B. in einer Home-Position).

Bewegungsdaten werden erhalten, in dem die von dem zumindest einem Bewegungssensor erfasste Bewegung überwacht wird. Das Überwachen der Bewegung ist breit zu verstehen. Das Überwachen kann eine Aufzeichnung und/oder eine Auswertung der erfassten Bewegung umfassen. Die Bewegungsdaten können direkt nach dem Festlegen der Ausgangsposition überwacht werden. Damit kann eine Position des Objektbereichs verfolgt werden. Die Bewegungsdaten können insbesondere auch dann gesammelt werden, während der Objektbereich in dem Untersuchungstunnel ist. Vorzugsweise werden die Bewegungsdaten zeitlich lückenlos überwacht nachdem die Ausgangsposition bestimmt wurde, zumindest bis zu einem Endzeitpunkt. Der Endzeitpunkt kann beispielsweise ein Ende einer Messung sein.

Mit den überwachten Bewegungsdaten kann eine aktuelle Position des Objektbereichs relativ zu der Ausgangsposition bestimmt werden. Die aktuelle Position kann optional auch eine aktuelle Orientierung umfassen. Vorteilhafterweise kann mit den Bewegungsdaten im Zusammenhang mit der Ausgangsposition eine aktuelle Position bestimmt werden. Beispielsweise können die Bewegungsdaten als relative Verschiebung zu der Ausgangsposition hinzuaddiert werden, um eine aktuelle Position des Objektbereichs zu ermitteln. Vorteilafterweise kann durch die Verwendung der Bewegungssensoren somit eine Bewegung auch noch in dem ansonsten für diese Zwecke schwer zugänglichen Untersuchungstunnel verfolgt werden. Eine Bewegung eines Patienten bzw. eines Teils des Patienten, insbesondere des Objektbereichs, kann somit erfasst und ggf. berücksichtigt werden. Die Bewegungsdaten bzw. die ermittelte aktuelle Position kann verwendet werden, um beispielsweise eine Bewegungskorrektur eines Scans aktuell oder retroaktiv vorzunehmen. Beispielsweise kann basieren auf den Bewegungsdaten bzw. der ermittelten aktuellen Position ein Sichtfeld (engl.: "Field of View") und/oder eine automatische Selektion angepasst werden. Vorteilhafterweise kann die Anpassung somit direkt vorgenommen werden und Bewegungen auch im Untersuchungstunnel relativ zuverlässig erfasst werden. Auch spontane Bewegungen, beispielsweise eines untersuchten Knies oder Ellbogens, können so detektiert und automatisch berücksichtigt werden. Insbesondere kann eine automatische Anpassung des Sichtfelds erfolgen und es ist tendenziell nicht mehr bzw. in der Regel nicht mehr notwendig, dass ein Nutzer ein manuelles Anpassen vornimmt.

Gemäß einer Ausführungsform sind zumindest drei Bewegungssensoren an der Trägereinheit befestigt. Die zumindest drei Bewegungssensoren können insbesondere zumindest drei Beschleunigungssensoren sein. Mit zumindest drei Bewegungssensoren kann eine besonders präzise und zuverlässige Bewegungsverfolgung möglich sein. Insbesondere kann auch ein Neigen oder Abknicken des Objektbereichs bestimmbar sein.

Gemäß einer Ausführungsform ist der zumindest eine Bewegungssensor zumindest ein Beschleunigungssensor, wobei die Bewegungsdaten Beschleunigungsdaten sind. Die aktuelle Position des Objektbereichs wird bestimmt, indem die Beschleunigungsdaten zweifach über die Zeit integriert werden. Ein Beschleunigungssensor kann eine besonders zuverlässige Möglichkeit sein, Bewegungsdaten auch innerhalb des Untersuchungstunnels zu bestimmen. Durch zweifaches Integrieren der erfassten Beschleunigung kann ein relativer Ort, d.h. eine Versetzung, des zumindest einen Beschleunigungssensors und damit auch des Objekts ermittelt werden. Die ermittelte Versetzung kann dann relativ zu der Ausgangsposition gesehen werden. Zusammen mit der Ausgangsposition kann somit die aktuelle Position bestimmt werden. Beschleunigungssensoren sind im Stand der Technik bereits bekannt und können für die Zwecke dieser Erfindung verwendet werden.

Gemäß einer Ausführungsform umfasst die Trägereinheit zusätzlich zumindest einen Magnetfeldsensor. Das Festlegen der Ausgangsposition umfasst:

- Anordnen des Objektbereichs mit der Trägereinheit außerhalb des Untersuchungstunnels;
- Bei eingeschaltetem Hauptmagneten des Magnetresonanztomographiesystems: Messen des Magnetfelds mit dem zumindest einen Magnetfeldsensor;
- Rückschließen auf die Position des Objektbereichs basierend auf dem gemessenen Magnetfeld.

Das Anordnen des Objektbereichs mit der Trägereinheit außerhalb des Untersuchungstunnels erfolgt insbesondere so, dass der Objektbereich vor einem Eingang des Untersuchungstunnels angeordnet wird, vorzugsweise auf einem Patiententisch. Der Magnetfeldsensor kann auf einer fest definierten Position und/oder mit einer fest definierten Orientierung auf der Trägereinheit angeordnet sein und/oder an der Trägereinheit befestigt sein. Der Magnetfeldsensor kann ein dreidimensionaler Magnetfeldsensor sein. Ein dreidimensionaler Magnetfeldsensor ist ein Magnetfeldsensor, der eine Magnetfeldstärke für jede von drei Raumrichtungen erfassen kann. Der Magnetfeldsensor kann beispielsweise ein Hall-Sensor sein. Beispielsweise können drei Hall-Elemente vorgesehen, sein, die jeweils eine Magnetfeldstärke in einer Raumrichtung bestimmen können. Alternativ können integrierte 3D-Hallsensoren verwendet werden. Mit 3D-Hallsensoren können insbesondere alle drei Raumrichtungen des Magnetfeldes bzw. der magnetischen Flussdichte von unterschiedlichen Abschnitten eines Sensor-Schaltkreises, insbesondere Sensor-ICs, erfasst werden. Dem Magnetfeldsensor kann ein Bewegungssensor zugeordnet sein. Vorzugsweise sind der Magnetfeldsensor und der zugeordnete Bewegungssensor zusammen auf einer starren Ebene der Trägereinheit angeordnet. Der zugeordnete Bewegungssensor kann insbesondere ein Beschleunigungssensor sein. Mit dem zugeordneten Bewegungssensor kann eine Positionsänderung des Magnetfeldsensors bzw. der Trägereinheit verfolgt werden, auch z.B. noch in dem Untersuchungstunnel. Der Bewegungssensor kann vorzugsweise neben oder auf oder unter dem Magnetfeldsensor angeordnet sein. Das Magnetfeld außerhalb des Untersuchungstunnels ist typischerweise inhomogen und abhängig von einem Abstand zu dem Untersuchungstunnel. Die Position des Magnetfeldsensors und somit die Position der Trägereinheit und somit die Position des Objektbereichs kann somit mit dem gemessenen Magnetfeld zusammenhängen. Grundsätzlich ist das Magnetfeld in der Regel geringer, je weiter der Abstand von dem Untersuchungstunnel ist. Mit Hilfe von Informationen über den Magnetfeldverlauf außerhalb des Magnetfeldtunnels kann somit im Zusammenhang mit dem gemessenen Magnetfeld auf die Position des Objektbereichs zurückgeschlossen werden. Es kann z.B. eine z-Position des Objektbereichs anhand des Magnetfelds bestimmt werden. Die z-Position kann aus einem Betrag des gemessenen Magnetfeldes bestimmt werden. Der Betrag ist insbesondere unabhängig von der Orientierung des Sensors. Beispielsweise können mit weiteren Sensoren (z.B. Beschleunigungssensoren) die x,y-Koordinaten ermittelt werden. Insbesondere können die Beschleunigungssensoren, verwendet werden um eine x-Koordinate und/oder eine y-Koordinate des Objektbereichs zu bestimmen. Die Bestimmung der x-Koordinate und/oder eine y-Koordinate kann mit Hilfe der Gravitation bestimmt werden. Beispielsweise kann zugrunde gelegt werden, dass eine x-Erstreckung horizontal ausgerichtet ist eine y-Erstreckung vertikal, d.h. parallel zu dem Gravitationsfeld der Erde, ausgerichtet ist. Mit einem oder mehreren weiteren Magnetfeldsensoren, z.B. zwei weiteren Magnetfeldsensoren, können weitere Positionsinformationen bestimmt werden. Die weiteren Magnetfeldsensoren können auf einer fest definierten Position auf der Trägereinheit angeordnet sein. Vorteilhafterweise kann durch die fest definierte Position eine relative Positionsbeziehung zu der Position zu dem ersten Magnetfeldsensor und zu der Trägereinheit festgelegt sein. Die weiteren Magnetfeldsensoren können dreidimensionale Magnetfeldsensoren sein. Die weiteren Magnetfeldsensoren können beispielsweise Hall-Sensoren sein. Einem oder mehreren, bevorzugt allen, der weiteren Magnetfeldsensoren kann jeweils zumindest ein Bewegungssensor zugeordnet sein. Vorzugsweise sind die weiteren Magnetfeldsensoren jeweils zusammen mit dem zugeordneten Bewegungssensor jeweils auf einer starren Ebene der Trägereinheit angeordnet. Mit den zugeordneten Bewegungssensoren kann eine Positionsänderung der Magnetfeldsensoren, insbesondere auch in dem Untersuchungstunnel, bestimmt werden. Die zugeordneten Bewegungssensoren können neben, auf oder unter dem jeweiligen Magnetfeldsensor angeordnet sein. Es kann vorgesehen sein, mehrere Ausgangspositionen zu bestimmen. Dazu kann es vorgesehen sein, die Position für mehrere Positionen der Patientenliege zu bestimmen und dazwischen die Patientenliege zu verfahren. Die Verwendung von mehreren Ausgangspositionen kann eine genauere Bestimmung der Position ermöglichen.

Gemäß einer Ausführungsform umfasst das Festlegen der Ausgangsposition weiter: Bestimmen einer Ausrichtung des Objektbereichs mit zumindest einem Orientierungssensor, insbesondere durch Messen einer durch die Gravitationskraft verursachten statischen Beschleunigung. Der Orientierungssensor kann insbesondere ein Beschleunigungssensor, der an der Trägereinheit angeordnet und/oder befestigt ist, sein. Mit einem Beschleunigungssensor kann die Orientierung im Erdgravitationsfeld bestimmt werden. Der Orientierungssensor kann einer der Bewegungssensoren bzw. der zumindest eine Bewegungssensor sein. Vorteilhafterweise kann beispielsweise mit einem Beschleunigungssensor sowohl eine Orientierung der Ausgangsposition bestimmt werden als auch eine Änderung der Position nach dem Bestimmen der Ausgangsposition. Der Orientierungssensor kann einem Magnetfeldsensor zugeordnet sein. Mit dem Orientierungssensor kann somit die Orientierung des Magnetfeldsensors bestimmt werden. Vorzugsweise sind der Magnetfeldsensor und der zugeordnete Orientierungssensor zusammen auf einer starren Ebene der Trägereinheit angeordnet. Optional kann ein externer Orientierungssensor vorgesehen sein, der nicht an der Trägereinheit angeordnet ist. Beispielsweise kann der Orientierungssensor auf einer optischen Erfassung basieren. Beispielsweise kann der Orientierungssensor eine Kamera sein, die dazu ausgestaltet ist, eine Orientierung der Trägereinheit und/oder des Magnetfeldsensors zu erfassen. Weil typischerweise das Magnetfeld außerhalb des Untersuchungstunnels rotationssymmetrisch um eine Achse des Untersuchungstunnels ist, kann anhand des Magnetfelds gegebenenfalls eine Orientierung nicht genau festgelegt werden. Hierbei kann mit dem Orientierungssensor abgeholfen werden, womit neben einer Position auch eine Orientierung des Magnetfeldsensors und/oder der Trägereinheit ermittelt werden kann. Damit kann letztlich auch eine Orientierung des Objektbereichs bestimmt werden.

Gemäß einer Ausführungsform wird die Trägereinheit gemäß einer definierten geometrischen Form, insbesondere zylinderförmig, um den Objektbereich herum angeordnet. Die Trägereinheit kann dazu ausgestaltet sein, die definierte geometrische Form automatisch anzunehmen oder die definierte geometrische Form zumindest teilweise unveränderlich aufweisen. Die definierte geometrische Form kann sich beispielsweise flexibel an den Objektbereich bzw. die Anatomie anpassen. Die definierte geometrische Form kann der Form des Objektbereichs entsprechen oder an die Form des Objektbereichs angepasst sein. Die Zylinderform kann beispielsweise durch Herumwickeln um den Objektbereich erzielt werden. Die Trägereinheit kann flächig ausgebildet sein und an die definierte geometrische Form anpassbar sein. Beispielsweise kann die Trägereinheit um ein Gelenk, insbesondere um ein Knie oder um einen Ellbogen, gewickelt werden. Die Trägereinheit kann beispielsweise schlauchförmig sein und durch Überziehen um den Objektbereich angeordnet werden. Durch die definierte geometrische Form kann eine Bewegungsbestimmung und/oder Bestimmung der Ausgangsposition besonders umfassend und präzise möglich sein. Insbesondere mit mehreren Bewegungssensoren können im Zusammenhang mit der definierten geometrischen Form auch komplexe Bewegungen erfassbar sein. Beispielsweise kann eine Zylinderform der Trägereinheit auch Drehungen von Gelenken, wie einem Knie oder einem Ellbogen, nachvollziehen.

Gemäß einer Ausführungsform ist zumindest ein zentraler Sensor, insbesondere umfassend einen zentralen Magnetfeldsensor und/oder einen zentralen Bewegungssensor, in Richtung einer Objektachse der geometrischen Form gesehen, insbesondere in Richtung einer Zylinderachse gesehen, zentral an dem Objektbereich angeordnet. Der zentrale Bewegungssensor kann insbesondere ein zentraler Beschleunigungssensor sein. Die Orientierung des Objektbereichs kann der Orientierung einer Achse des zentralen Beschleunigungssensors entsprechen, wobei die Achse insbesondere entlang der Zylinderachse verläuft. Der zentrale Bewegungssensor kann beispielsweise dazu verwendet werden, eine Orientierung einer Längsachse des Objektbereichs, die insbesondere der Zylinderachse entsprechen kann, zu bestimmen bzw. deren Änderung zu verfolgen. Zusätzlich oder alternativ kann eine Positionsbestimmung des Objektbereichs mit dem zentralen Bewegungssensor bestimmt und/oder unterstützt werden. Beispielsweise kann mit dem zentralen Magnetfeldsensor eine Ausgangsposition des Objektbereichs, insbesondere besonders genau, bestimmt werden.

Gemäß einer Ausführungsform wird die aktuelle Position des Objektbereichs bestimmt, indem ein Kreis, der durch das Zentrum des Objektbereichs verläuft und einem Querschnitt der geometrischen Form, insbesondere des Zylinders, entspricht, konstruiert wird, wobei der Kreis mit Hilfe von Sensordaten des zentralen Sensors und mit Sensordaten von zwei weiteren Sensoren, insbesondere von zwei weiteren Magnetfeldsensoren und/oder Bewegungssensoren, die auf die Ebene des Kreises projiziert werden, konstruiert wird. Die zwei weiteren Magnetfeldsensoren können Hall-Sensoren sein. Die zwei weiteren Magnetfeldsensoren können insbesondere dreidimensionale Magnetfeldsensoren sein. Die zwei weiteren Bewegungssensoren können insbesondere Beschleunigungssensoren sein, insbesondere können die zwei weiteren Bewegungssensoren und der zentrale Bewegungssensor Beschleunigungssensoren sein. Die Beschleunigungssensoren können dazu verwendet werden eine Orientierung der Magnetfeldsensoren zu bestimmen. Die Bewegungssensoren bzw. Beschleunigungssensoren könnend dazu verwendet werden eine Bewegungsverfolgung der Magnetfeldsensoren zu erzielen. Zum Bestimmen des Kreises kann beispielsweise davon ausgegangen werden, dass die drei Sensoren auf einer Ellipse liegen. Unter der Annahme, dass der zentrale Sensor bereits auf dem Kreis liegt (da sowohl der zentrale Sensor als auch der Kreis im zentral an dem Objektbereich sind) können die Positionen der weiteren Sensoren mit bekannten geometrischen Überlegungen auf den Kreis projiziert werden. Vorteilhafterweise kann mit den Sensoren der Kreis somit definiert und somit auch lokalisiert werden. Mit Hilfe des Kreises kann wiederum auf die Position und Orientierung des Objektbereichs rückgeschlossen werden.

Gemäß einer Ausführungsform wird eine Position des Objektbereichs über den Kreismittelpunkt bestimmt. Da der Kreis zentral auf der Längsachse des Objektbereichs liegt und zudem zumindest näherungsweise dem Querschnitt des Objektbereichs entsprechen kann, ist es möglich mit über den Kreismittelpunkt auch den Mittelpunkt des Objektbereichs zu bestimmen. Während in dem Mittelpunkt des Objektbereichs selbst in der Regel kein Sensor platziert werden kann, weil diese (z.B. im Inneren eines Knies) in der Regel dafür nicht zugänglich ist, kann mit der Konstruktion eines Kreises und mit Hilfe der zumindest drei Sensoren eine Position des Mittelpunkts des Objektbereichs konstruiert werden.

Gemäß einer Ausführungsform wird eine Größe des Objektbereichs über einen Radius des Kreises bestimmt. Vorteilhafterweise kann somit über den automatisch bestimmbaren Kreis auch die Größe des Objektbereichs ermittelt werden. Dies kann insbesondere vorteilhaft sein bei größenveränderlichen Objekten, aber auch um die Größe als Basis überhaupt festzulegen. Optional kann auch eine Ausgangsgröße entsprechend der Ausgangsposition bestimmt werden. In dem Fall kann auch eine Größenverfolgung analog zu der Bewegungsverfolgung möglich sein.

Gemäß einer Ausführungsform wird eine Orientierung des Objektbereichs anhand einer Achse des zumindest einen zentralen Sensors bestimmt, die in einer definierten Orientierung, insbesondere parallel, zu einer Objektachse, insbesondere Zylinderachse, verläuft. Die Objektachse ist insbesondere eine Objektachse des Objektbereichs. Ist der Objektbereich beispielsweise (näherungsweise) zylinderförmig, so kann die Objektachse der Achse des Zylinders entsprechen. Die Objektachse des Objektbereichs kann einer Achse der definierten geometrischen Form der Trägereinheit entsprechen, insbesondere der Zylinderachse einer Zylinderform der Trägereinheit. Die Orientierung der Achse des zentralen Sensors relativ zu der geometrischen Form kann durch eine Befestigung des Bewegungssensors an der Trägereinheit festgelegt sein. Durch geeignetes Anordnen der Trägereinheit um den Objektbereich herum kann die Achse der Trägereinheit zu der Objektachse ausgerichtet werden. Beispielsweise kann die Achse der Trägereinheit eine Zylinderachse einer zylinderförmig um den Objektbereich herum angeordneten Trägereinheit sein. In diesem Beispiel ist die geometrische Form der Trägereinheit ein Zylinder. Somit kann die Achse des Bewegungssensors mit der Ausrichtung des Objektbereichs verknüpft sein. Der zumindest eine zentrale Sensor kann insbesondere zwei weitere Achsen aufweisen, die senkrecht zu der ersten Achse ist. Im Fall einer zylinderförmigen Trägereinheit können die zwei weiteren Achsen des zentralen Sensors insbesondere in einer Ebene liegen, welche den Zylinder ellipsenförmig schneiden. Insbesondere können bei zumindest zwei weiteren Sensoren, die zusätzlich zu dem zumindest einen zentralen Sensor vorgesehen sind, die Achsen der weiteren Sensoren eine definierte Beziehung zu der Achse des zentralen Sensors aufweisen. Die weiteren Sensoren können weitere Magnetfeldsensoren und/oder Bewegungssensoren wie hierin beschrieben sein.

Ein weiterer Aspekt der Erfindung ist eine Messvorrichtung umfassend eine Trägereinheit, insbesondere Magnetresonanztomographie-Körperspule, und zumindest einen Bewegungssensor, insbesondere Beschleunigungssensor, bevorzugt zumindest drei Bewegungssensoren, wobei der zumindest eine Bewegungssensor an der Trägereinheit befestigt ist, wobei die Trägereinheit im Wesentlichen zylinderförmig ist oder in eine Zylinderform formbar ist. Der Bewegungssensor kann insbesondere ein dreidimensionaler Bewegungssensor sein. Der Beschleunigungssensor kann insbesondere ein dreidimensionaler Beschleunigungssensor sein. Alle Vorteile und Merkmale des Verfahrens können analog auf die Messvorrichtung übertragen werden und umgekehrt.

Gemäß einer Ausführungsform umfasst die Messvorrichtung einen oder mehrere Magnetfeldsensoren, die an der Trägereinheit befestigt sind. Die Magnetfeldsensoren können vorzugsweise dreidimensionale Magnetfeldsensoren sein. Die Magnetfeldsensoren können Hall-Sensoren sein. Beispielsweise können drei Magnetfeldsensoren vorgesehen sein. Vorzugsweise ist zumindest ein Magnetfeldsensor, insbesondere zumindest ein zentraler Magnetfeldsensor, zusammen mit zumindest einem Orientierungssensor, insbesondere Beschleunigungssensor, auf einer starren Ebene der Trägereinheit angeordnet. Die starre Ebene kann beispielsweise eine Platine sein. Die Platine kann eine Platine einer Körperspule sein, insbesondere, wenn die Trägereinheit eine Körperspule ist.

Gemäß einer Ausführungsform weist die Zylinderform der Trägereinheit oder die Zylinderform, in die die Trägereinheit formbar ist, eine Zylinderachse auf, wobei zumindest einer der Sensoren, insbesondere ein Magnetfeldsensor und/oder Bewegungssensor, in Richtung der Zylinderachse gesehen im Wesentlichen zentral auf der Trägereinheit angeordnet ist. Vorteilhafterweise kann mit einer zentralen Anordnung eine Positionsbestimmung erleichtert werden. Insbesondere kann es vorgesehen sein, dass der zentrale Sensor zentral auf dem Objektbereich angeordnet wird.

Gemäß einer Ausführungsform ist die Trägereinheit starr gegenüber einer Verformung fixiert und/oder fixierbar in der Zylinderform. Vorteilhaferweise kann somit eine Verformung der Trägereinheit und auch eine Verzerrung des Objektbereichs während eines Scans verhindert werden, womit die Bewegungsverfolgung erleichtert werden kann.

Gemäß einer Ausführungsform weist die Trägereinheit eine Markierung auf, die zumindest eine definierte, insbesondere die zentrale, Position auf der Zylinderachse markiert. Vorteilhafterweise kann mit der Markierung eine exakte Positionierung der Trägereinheit an/auf dem Objektbereich erleichtert werden. Beispielsweise kann die Markierung ein Kreuz sein oder umfassen. Die Markierung kann ein Muster umfassen, dass entsprechend dem Objektbereich und/oder entsprechend einer vorgesehenen Positionierung um den Objektbereich herum ausgestaltet ist. Beispielsweise kann durch die Markierung eine Längserstreckung definiert sein, die entlang einer Längserstreckung des Objektbereichs ausgerichtet wird. Beispielsweise kann die Längserstreckung entlang einem Ellbogen ausgerichtet werden. Die Längserstreckung kann beispielsweise durch einen länglichen Kasten und/oder eine gerade Linie definiert sein. Die Markierung kann einen Maßstab umfassen. Der Maßstab kann dazu ausgestaltet sein, dass eine Erstreckung des Objektbereichs anhand des Maßstabs abgelesen werden kann.

Gemäß einer Ausführungsform weist die Messvorrichtung weitere Sensorelemente auf. Die weiteren Sensorelemente können beispielsweise dazu ausgestaltet sein, weitere Informationen zu erfassen. Weitere Informationen können beispielsweise weitere Positionsinformationen, eine Temperatur etc. sein. Beispielsweise können MR-Marker vorgesehen sein. Zusätzlich oder alternativ können an der Trägereinheit ein oder mehrere Schildelemente, z.B. elektromagnetische Abschirmungen, vorgesehen sein.

Optional können an der Trägereinheit kleine Sende- und/oder Empfang-Spulen (TX- und/oder RX-Spulen) angeordnet sein. Die Spulen können relativ zu zumindest einer Markierung angeordnet sein. Die Spulen können um eine Markierung herumgewickelt sein.

Ein weiterer Aspekt der Erfindung ist ein Magnetresonanztomographiesystem umfassend eine Messvorrichtung wie hierin beschrieben und einen Untersuchungstunnel, wobei das Magnetresonanztomographiesystem dazu konfiguriert ist, mit dem zumindest einen Bewegungssensor die Bewegung eines Objektbereichs in dem Untersuchungstunnel zu verfolgen, insbesondere gemäß einem Verfahren wie hierin beschrieben. Alle Vorteile und Merkmale des Verfahrens und der Messvorrichtung können analog auf das Magnetresonanztomographiesystem übertragen werden und umgekehrt.

Alle hierin beschriebenen Ausführungsformen können miteinander kombiniert werden, soweit nicht explizit etwas anderes angegeben ist.

Im Folgenden werden Ausführungsformen mit Bezug auf die beigefügten Figuren beschrieben.

Fig. 1     zeigt ein Flussdiagramm eines Verfahrens zur Bewegungsverfolgung eines Objektbereichs in einem Untersuchungstunnel eines Magnetresonanztomographiesystems gemäß einer Ausführungsform der Erfindung,

Fig. 2     zeigt ein Flussdiagramm eines Verfahrens zur Bewegungsverfolgung eines Objektbereichs in einem Untersuchungstunnel eines Magnetresonanztomographiesystems gemäß einer weiteren Ausführungsform der Erfindung,

Fig. 3     zeigt eine Messvorrichtung mit einer Trägereinheit gemäß einer Ausführungsform der Erfindung,

Fig. 4     zeigt die Messvorrichtung gemäß der Ausführungsform von Figur 3 in einem ausgebreiteten Zustand und in einem zu einem Zylinder gewickelten Zustand,

Fig. 5     zeigt ein Magnetresonanztomographiesystem mit einer Messvorrichtung gemäß einer Ausführungsform der Erfindung,

Fig. 6     zeigt ein Magnetresonanztomographiesystem mit einer Messvorrichtung gemäß der Ausführungsform in Figur 5, und

Fig. 7     illustriert eine Möglichkeit der Berechnung der aktuellen Position, inklusive der Orientierung, des Objektbereichs aus Sensordaten gemäß einer Ausführungsform der Erfindung.

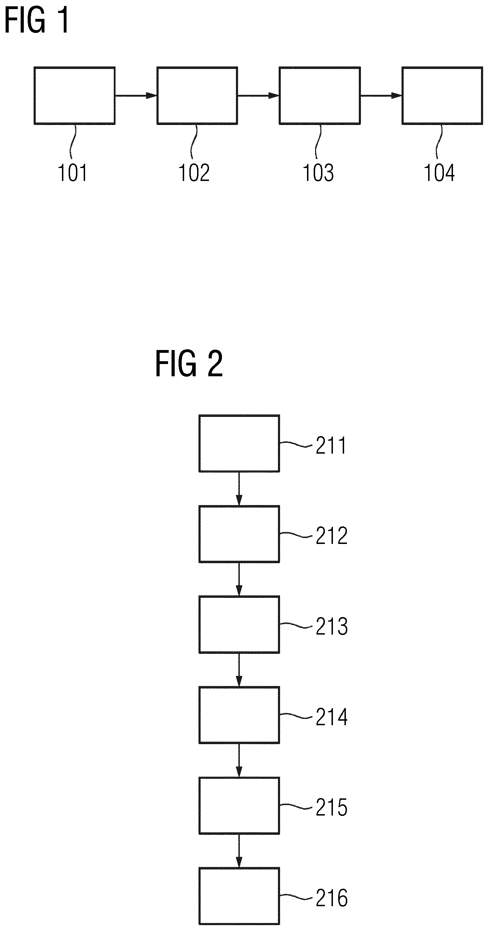

Figur 1 zeigt ein Flussdiagramm eines Verfahrens zur Bewegungsverfolgung eines Objektbereichs in einem Untersuchungstunnel 6 eines Magnetresonanztomographiesystems gemäß einer Ausführungsform der Erfindung. In einem ersten Schritt 101 wird eine Trägereinheit 1 um den Objektbereich herum angeordnet, sodass die Position der Trägereinheit 1 mit der Position des Objektbereichs verknüpft ist. Die Trägereinheit 1 kann optional eine Magnetresonanztomographie-Körperspule sein. Vorteilhafterweise kann in diesem Fall die Trägereinheit 1 sowohl für den MRT-Messprozess an sich als auch für die Bewegungsbestimmung bzw. Bewegungsverfolgung verwendet werden. An der Trägereinheit 1 ist zumindest ein Bewegungssensor angeordnet. Vorzugsweise können mehrere Bewegungssensoren, insbesondere drei Bewegungssensoren, an der Trägereinheit 1 angeordnet sein. Mehrere Bewegungssensoren können eine detailliertere Bewegungsverfolgung ermöglichen. Der Bewegungssensor kann bzw. die Bewegungssensoren können vorzugsweise ein Beschleunigungssensor 3 bzw. Beschleunigungssensoren 3 sein. In einem weiteren Schritt 102 wird eine Ausgangsposition des Objektbereichs festgelegt. Die Ausgangsposition kann optional eine Ausgangsorientierung umfassen. Die Ausgangsposition kann beispielsweise festgelegt werden, indem sie von einem Nutzer eingegeben wird, von einem externen Gerät empfangen wird und/oder von einer Sensorik ermittelt wird. In einem weiteren Schritt 103 wird die von dem zumindest einen Bewegungssensor erfasste Bewegung überwacht, um Bewegungsdaten zu erhalten. Ist der zumindest eine Bewegungssensor ein Beschleunigungssensor 3 so können die Bewegungsdaten Beschleunigungsdaten des Beschleunigungssensors 3 sein. Mit anderen Worten kann eine Beschleunigung des Objektbereichs, vorzugsweise durchgehend, detektiert und protokolliert werden. In einem weiteren Schritt 104 wird eine aktuelle Position des Objektbereichs relativ zu der Ausgangsposition bestimmt indem die mit dem zumindest einen Bewegungssensor erfassten Bewegungsdaten verwendet werden. Die aktuelle Position kann vorzugsweise auch eine aktuelle Orientierung umfassen. Damit kann auf die absolute aktuelle Position des Objektbereichs geschlossen werden. Werden ein oder mehrere Beschleunigungssensoren 3 verwendet, so kann die aktuelle Position bestimmt werden, indem die Beschleunigungsdaten zweifach über die Zeit integriert werden.

Figure 2 zeigt ein Flussdiagramm eines Verfahrens zur Bewegungsverfolgung eines Objektbereichs in einem Untersuchungstunnel 6 eines Magnetresonanztomographiesystems gemäß einer weiteren Ausführungsform der Erfindung. In einem ersten Schritt 211 wird eine Trägereinheit 1 um den Objektbereich herum angeordnet, sodass die Position der Trägereinheit 1 mit der Position des Objektbereichs verknüpft ist. Die Trägereinheit 1 kann optional eine Magnetresonanztomographie-Körperspule sein. An der Trägereinheit 1 sind drei Beschleunigungssensoren 3 sowie drei Magnetfeldsensoren 5 angeordnet. Dabei ist jeweils ein Beschleunigungssensor 3 jeweils einem Magnetfeldsensor 5 zugeordnet, sodass es drei Paare aus je einem Beschleunigungssensor 3 und einem Magnetfeldsensor 5 gibt. Die Paare sind jeweils auf einer starren Fläche der Trägereinheit 1 angeordnet. Dadurch kann sichergestellt werden, dass die Orientierung der Sensorpaare zueinander konstant ist. In einem weiteren Schritt 212 wird der Objektbereich mit der Trägereinheit 1 außerhalb des Untersuchungstunnels 6 auf einer Patientenliege 7 vor dem Eingang des Untersuchungstunnels 6 angeordnet. In einem weiteren Schritt 213, während der Objektbereich mit der Trägereinheit 1 noch außerhalb des Untersuchungstunnels 6 ist, wird bei eingeschaltetem Hauptmagneten des Magnetresonanztomographiesystems das Magnetfeld mit den Magnetfeldsensoren 5 gemessen. Zudem wird mit den Beschleunigungssensoren 3 eine Orientierung im Gravitationsfeld der Erde gemessen. Die Beschleunigungssensoren 3 fungieren in diesem Fall als Orientierungssensoren. In einem weiteren Schritt 214 wird die Ausgangsposition des Objektbereichs basierend auf dem gemessenen Magnetfeld ermittelt. Indem das Magnetfeld außerhalb des Untersuchungstunnels 6 von dem Abstand zu dem Untersuchungstunnel 6 abhängt, kann eine z-Position, d.h. eine Entfernung von dem Untersuchungstunnel 6 entlang der Mittelachse des Untersuchungstunnels 6 bestimmt werden. Durch das Verwenden von mehreren Magnetfeldsensoren 5 kann zudem ein Abstand von der Mittelachse des Untersuchungstunnels 6 ermittelt werden. Weil das Magnetfeld außerhalb des Untersuchungstunnels 6 typischerweise rotationssymmetrisch ist, kann es ggf. schwierig oder unmöglich sein, allein anhand der Magnetfeldmessung auch eine exakte Orientierung zu bestimmen. Hierfür werden die Daten von den Beschleunigungssensoren 3 verwendet, mit denen eine Ausrichtung im Gravitationsfeld der Erde ermittelt werden kann. Durch die Beschleunigungssensoren 3 kann den Magnetfeldsensoren 5 auch eine absolute Orientierung zugeordnet werden, wodurch insgesamt sowohl die Koordinatenposition als auch die Orientierung der Trägereinheit 1 und damit des Objektbereichs bestimmt werden kann. Die ermittelte Ausgangsposition umfasst somit nicht nur eine Koordinatenposition, sondern auch eine Orientierung im Raum. In einem weiteren Schritt 215 wird die von den Beschleunigungssensoren 3 erfasste Bewegung in Form von Beschleunigungsdaten überwacht, und die Beschleunigungsdaten werden aufgezeichnet bzw. im nächsten Schritt direkt weiter ausgewertet. Die Beschleunigungsdaten werden von den Beschleunigungssensoren 3 erfasst. Somit können die Beschleunigungssensoren 3 nicht nur zur Ermittlung einer Orientierung der Ausgangsposition, sondern auch zu einer Verfolgung der Bewegung, insbesondere auch im Untersuchungstunnel 6 verwendet werden. Vorzugsweise wird so eine Beschleunigung des Objektbereichs durchgehend detektiert und protokolliert. In einem weiteren Schritt 216 wird eine aktuelle Position des Objektbereichs relativ zu der Ausgangsposition bestimmt, indem die mit Beschleunigungssensoren 3 erfassten Beschleunigungsdaten verwendet werden. Dazu werden die Beschleunigungsdaten zweifach über die Zeit integriert, um auf die Verschiebung des Ortes der einzelnen Sensoren zu schließen. Damit kann auf die absolute aktuelle Position des Objektbereichs geschlossen werden. Die aktuelle Position kann vorzugsweise auch eine aktuelle Orientierung umfassen. Aus den ermittelten aktuellen Positionen der Sensoren kann sowohl eine aktuelle Koordinatenposition als auch eine aktuelle Orientierung im Raum des Objektbereichs ermittelt werden, indem die relative Position der Sensoren zueinander berücksichtigt wird.

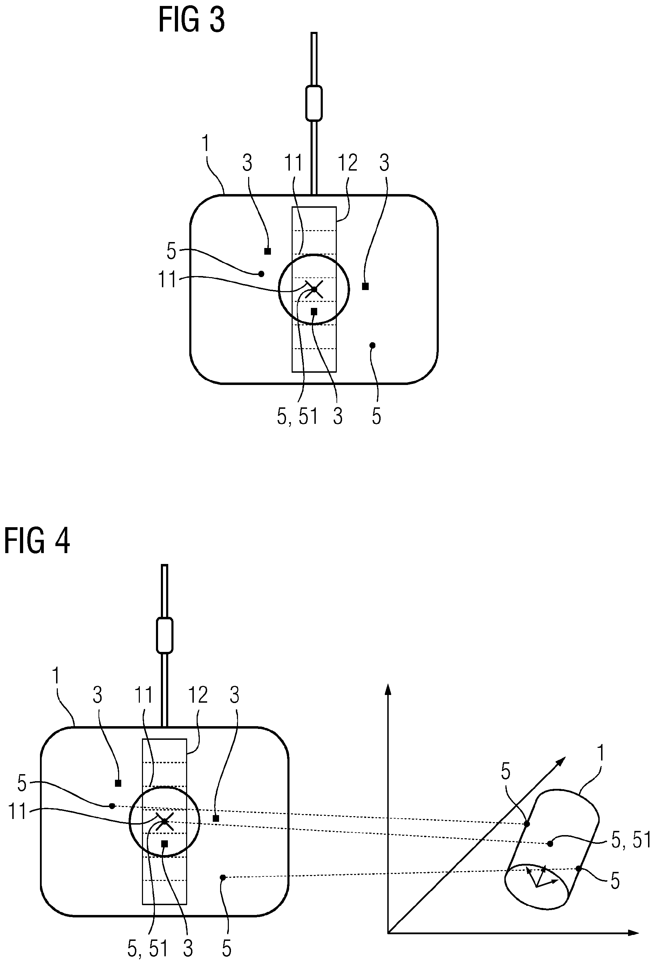

Figur 3 zeigt eine Messvorrichtung mit einer Trägereinheit 1 gemäß einer Ausführungsform der Erfindung. Die Trägereinheit 1 kann insbesondere gleichzeitig als MRT-Körperspule dienen. Auf der Trägereinheit 1 sind je drei Beschleunigungssensoren 3 und drei Magnetfeldsensoren 5 angeordnet und befestigt. Die Trägereinheit 1 weist eine Markierung auf, die eine zentrale Position auf der Zylinderachse markiert. Die Markierung umfasst in diesem Ausführungsbeispiel ein Kreuz 11. Das Kreuz 11 dient als Orientierung, um zu gewährleisten, dass die Trägereinheit 1 richtig bzw. an der richtigen Stelle um den Objektbereich herum angeordnet wird. Insbesondere ist es vorgesehen, dass die Stelle mit dem Kreuz 11 über einem Zentrum des Objektbereichs platziert wird. Beispielsweise kann das Kreuz 11 direkt an einem Knie oder Ellbogen platziert werden. Weiterhin umfasst die Markierung eine Skala 12, welche zur Ausrichtung an einem länglichen Objektbereich, wie einem Ellbogen- oder einem Kniebereich, dienen kann und mit der zudem eine Länge des Objektbereichs abgemessen werden kann. Beispielsweise kann die Längserstreckung der Skala 12 an einer Längserstreckung eines Arms oder eines Beins ausgerichtet werden. Mit der Markierung kann somit eine korrekte definierte Ausrichtung und Positionierung der Trägereinheit 1 relativ zu dem Objektbereich gefördert werden.

In Figur 4 ist die Messvorrichtung mit einer Trägereinheit 1 gemäß der Ausführungsform von Figur 3 in einem ausgebreiteten Zustand und in einem zu einem Zylinder gewickelten Zustand gezeigt. In der Zylinderform kann die Trägereinheit 1 insbesondere um einen Objektbereich herum angeordnet sein. Die Trägereinheit 1 ist so ausgestaltet, dass sie in der Zylinderform starr fixierbar ist, sodass eine wesentliche Verformung der Trägereinheit 1 während eines MRT-Scans verhindert ist. Einer der Magnetfeldsensoren 5 ist ein zentraler Magnetfeldsensor 51, der im Wesentlichen zentral auf der Zylinderachse der Trägereinheit 1 angeordnet ist.

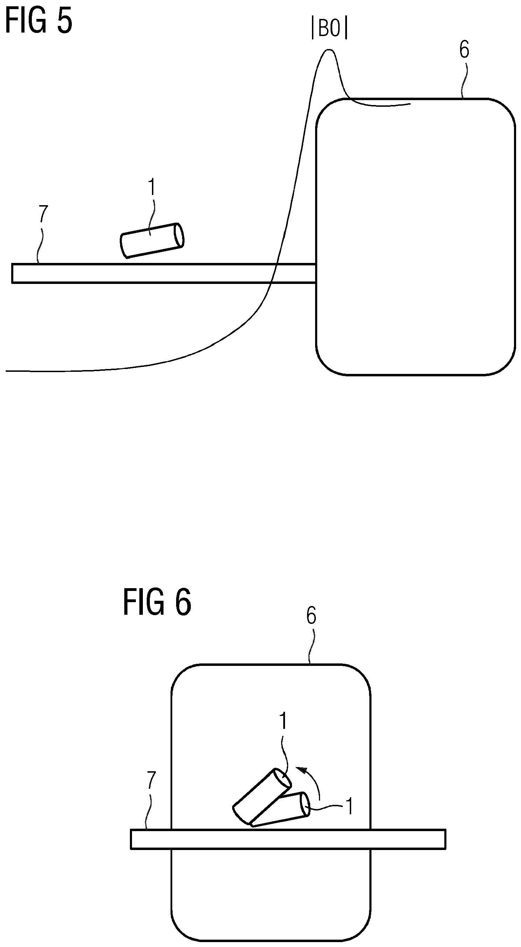

Die Figuren 5 und 6 zeigen ein Magnetresonanztomographiesystem mit einer Messvorrichtung gemäß einer Ausführungsform der Erfindung. Zu sehen ist die Trägereinheit 1 der Messvorrichtung. Die Sensoren der Messvorrichtung sind in dieser Darstellung nicht eingezeichnet, sie können beispielsweise denjenigen entsprechen, wie sie in den Figuren 3 und 4 gezeigt sind. Das Magnetresonanztomographiesystem ist dazu konfiguriert, mit den Bewegungssensoren, beispielsweise Beschleunigungssensoren 3, die an der Trägereinheit 1 befestigt sind die Bewegung eines Objektbereichs in einem Untersuchungstunnel 6 des Magnetresonanztomographiesystems zu verfolgen. In Figur 5 ist die Trägereinheit 1 außerhalb des Untersuchungstunnels 6 um einen hier nicht gezeigten Objektbereich herum angeordnet. Insbesondere liegt dabei ein Patient auf einer Patientenliege 7, und der Objektbereich kann ein Körperteil des Patienten sein. Diese Position kann als Ausgangsposition bzw. Home-Position der Patientenliege 7 verstanden werden. Die Trägereinheit 1 ist hier bereits um den (nicht gezeigten) Objektbereich herumgewickelt. In dieser Position kann bei eingeschaltetem Hauptmagneten des Magnetresonanztomographiesystems das Magnetfeld (B0) von den Magnetfeldsensoren 5 an der Position der Magnetfeldsensoren 5 gemessen. Anhand der Größe des Magnetfelds bei den verschiedenen Magnetfeldsensoren 5 kann auf eine Positionierung der Trägereinheit 1 und des Objektbereichs in dieser Ausgangsposition geschlossen werden. Weil das Magnetfeld in der Regel näherungsweise rotationssymmetrisch um eine Achse des Untersuchungstunnels 6 ist, wird zur Bestimmung der Orientierung der Ausgangsposition zumindest ein Orientierungssensor verwendet. Der Orientierungssensor kann ein Beschleunigungssensor 3 sein, mit dem die Erdbeschleunigung gemessen und damit die Orientierung im Erdmagnetfeld bestimmt werden kann.

Wird dann die Patientenliege 7 mit dem Objektbereich und der Trägereinheit 1 in den Untersuchungstunnel 6 verfahren, wie in Figur 6 gezeigt, so ist eine Messung des Magnetfelds wenig hilfreich für die weitere Positionsbestimmung, weil das Magnetfeld in dem Untersuchungstunnel 6 weitgehend homogen ist. Um die Bewegung weiterzuverfolgen, werden nun die Beschleunigungssensoren 3 oder andere Bewegungssensoren verwendet. Durch Messen der Beschleunigung mit Beschleunigungssensoren 3 kann durch zweifache Integration auf die Ortsveränderung geschlossen werden. Vorzugsweise werden dazu zeitlich lückenlos Beschleunigungsdaten aufgenommen. Wird somit die Ortsveränderung zu der Ausgangsposition hinzuaddiert, so kann die jeweils aktuelle Position bestimmt werden. In Figur 6 sind andeutungsweise eine Bewegung (entlang des Pfeils) und entsprechend zwei unterschiedliche Positionen der Trägereinheit 1 eingezeichnet. Mit den gesammelten Bewegungsdaten kann beispielsweise ermittelt werden, ob eine Ausrichtung eines Sichtfelds des Scans aufgrund einer Bewegung des untersuchten Objektbereichs angepasst werden muss.

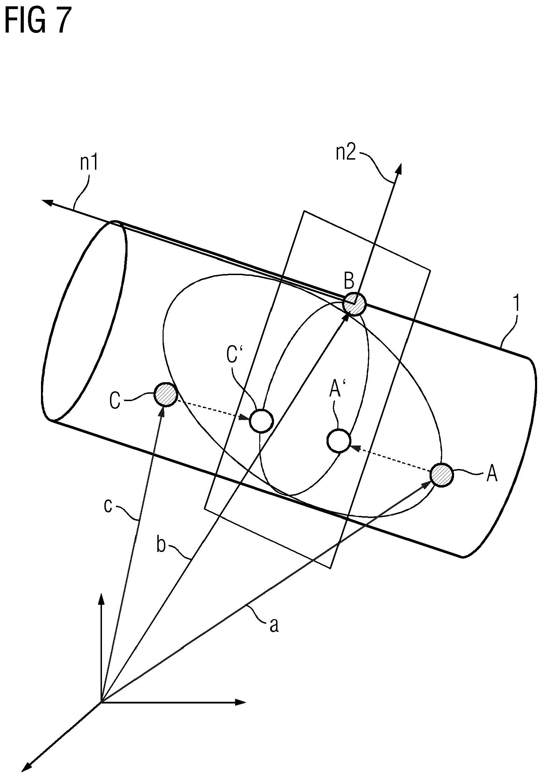

Figur 7 illustriert eine Möglichkeit der Berechnung der aktuellen Position, inklusive der Orientierung, des Objektbereichs gemäß einer Ausführungsform der Erfindung. Die Position des Objektbereichs kann anhand der Position von drei Sensoren, die auf der Trägereinheit 1 angeordnet und befestigt sind, bestimmt werden. Die drei Sensoren können beispielsweise Magnetfeldsensoren 5 sein. Die Magnetfeldsensoren 5 können mit je einem Beschleunigungssensor 3 verknüpft sein, um einerseits die Orientierung der Magnetfeldsensoren 5 außerhalb des Untersuchungstunnels 6 genauer zu bestimmen und andererseits die Bewegung der Magnetfeldsensoren 5 auch innerhalb des Untersuchungstunnels 6 nachverfolgen zu können. Die jeweils aktuelle Position ($a$, $b$, $c$) der Sensoren kann basierend auf der jeweiligen Ausgangsposition ($A$, $B$, $C$) durch einfache Addition mit der registrierten Bewegung ($D\_A$, $D\_B$, $D\_C$) bestimmt werden:

$$a = A + D\_A$$

$$b = B + D\_B$$

$$c = C + D\_C$$

Die Bewegung ($D\_A$, $D\_B$, $D\_C$) kann bei Verwendung von Beschleunigungssensoren 3 jeweils durch zweifache Integration über die erfassten Beschleunigungsdaten ermittelt werden. Sobald die aktuelle Position der drei Sensoren bestimmt ist, kann die Position inklusive der Orientierung der zylinderförmigen Trägereinheit 1 bestimmt werden, womit wiederum direkt auf die Position des Objektbereichs geschlossen werden kann. In Figur 7 ist dazu die zylinderförmige Trägereinheit 1 mit den Sensoren A, B, C gezeigt, wobei die aktuelle Position der Sensoren durch die Vektoren a, b, c definiert ist. Die Sensoren A, B, C liegen auf einer Ebene, welche den Zylinder in Gestalt einer Ellipse schneidet. Die Sensoren A, B, C liegen zudem auf der Oberfläche des Zylinders. Der zentrale Sensor B, der, in Richtung der Zylinderachse gesehen, zentral mittig angeordnet ist, ist so ausgerichtet, dass eine erste Achse n1 des zentralen Sensors mit der Orientierung des Zylinders übereinstimmt bzw. parallel zu der Zylinderachse ausgerichtet ist. Eine von den anderen beiden Achsen (n2, n3) des zentralen Sensors aufgespannte Ebene weist einen kreisförmigen Schnitt mit dem Zylinder auf, der dem kreisförmigen Querschnitt des Zylinders an der Position des zentralen Sensors entspricht. Zur Berechnung von Position und Größe des Kreises können die Positionen (A, C) der anderen beiden Sensoren entlang der Achse n1 auf die Ebene des Kreises projiziert werden, wodurch die auf dem Kreisumfang liegenden Punkte A' und C' erhalten werden mit den Vektoren a' und c' erhalten werden (mit den Geraden-Gleichungen $a + \lambda\_A \times n1$ und $c + \lambda\_C \times n1$):

```
a' = Schnitt_der_Ebene_mit_der_Geraden(Ebene(n2,n3), Gerade(a
+ λ_A×n1))
```

```
c' = Schnitt_der_Ebene_mit_der_Geraden (Ebene (n2,n3), Gerade
(c + λ_C×n1))
```

Mit den Koordinaten der Punkte (a', b, c') auf dem Kreisumfang können wiederum die Parameter des Kreises bestimmt werden, umfassend das Zentrum des Kreises und dessen Radius. Damit ist Position und Größe des Objektbereichs bestimmt. Die Position des Objektbereichs entspricht der Position des Kreismittelpunkts. Die Orientierung des Objektbereichs ist durch n1 definiert. Die Größe des Objektbereichs ist im Querschnitt durch den Radius des Kreises bestimmt und kann in der Längsrichtung beispielsweise mit Hilfe einer Markierung an der Trägereinheit 1 oder auf andere Weise abgemessen werden oder aus beispielsweise aus einer Datenbank abgerufen werden. Alternativ kann die Erstreckung in Längsrichtung auch beispielsweise mit einem statistischen Körpermodell ermittelt werden (beispielsweise basierend auf einer Körpergröße des Patienten, der untersucht wird), wie es im Stand der Technik bekannt ist.

**Patentansprüche**

1.  Verfahren zur Bewegungsverfolgung eines Objektbereichs in einem Untersuchungstunnel (6) eines Magnetresonanztomographiesystems, wobei das Verfahren die folgenden Schritte umfasst:

    (a) Anordnen einer Trägereinheit (1) um den Objektbereich herum, sodass die Position der Trägereinheit (1) mit der Position des Objektbereichs verknüpft ist, wobei die Trägereinheit (1) insbesondere eine Magnetresonanztomographie-Körperspule ist, wobei an der Trägereinheit (1) zumindest ein Bewegungssensor angeordnet ist;
    (b) Festlegen einer Ausgangsposition des Objektbereichs;
    (c) Überwachen der von dem zumindest einen Bewegungssensor erfassten Bewegung, um Bewegungsdaten zu erhalten;
    (d) Bestimmen einer aktuellen Position des Objektbereichs relativ zu der Ausgangsposition basierend auf den mit dem zumindest einen Bewegungssensor erfassten Bewegungsdaten.

2.  Verfahren gemäß Anspruch 1,

    wobei der zumindest eine Bewegungssensor zumindest ein Beschleunigungssensor (3) ist, wobei die Bewegungsdaten Beschleunigungsdaten sind;
    wobei die aktuelle Position des Objektbereichs bestimmt wird, indem die Beschleunigungsdaten zweifach über die Zeit integriert werden.

3.  Verfahren gemäß einem der vorhergehenden Ansprüche, wobei die Trägereinheit (1) zusätzlich zumindest einen Magnetfeldsensor (5) umfasst;
    wobei das Festlegen der Ausgangsposition umfasst:

    - Anordnen des Objektbereichs mit der Trägereinheit (1) außerhalb des Untersuchungstunnels (6);
    - Bei eingeschaltetem Hauptmagneten des Magnetresonanztomographiesystems: Messen des Magnetfelds mit dem zumindest einen Magnetfeldsensor (5);
    - Rückschließen auf die Position des Objektbereichs basierend auf dem gemessenen Magnetfeld.

4.  Verfahren gemäß Anspruch 3,
    wobei das Festlegen der Ausgangsposition weiter umfasst:

- Bestimmen einer Ausrichtung des Objektbereichs mit zumindest einem Orientierungssensor, insbesondere durch Messen einer durch die Gravitationskraft verursachten statischen Beschleunigung, wobei der zumindest eine Bewegungssensor den zumindest einen Orientierungssensor, insbesondere zumindest einen Beschleunigungssensor (3), umfasst.

5. Verfahren gemäß einem der vorhergehenden Ansprüche, wobei die Trägereinheit (1) gemäß einer definierten geometrischen Form, insbesondere zylinderförmig, um den Objektbereich herum angeordnet wird.

6. Verfahren gemäß Anspruch 5,
wobei zumindest ein zentraler Sensor, insbesondere umfassend einen zentralen Magnetfeldsensor (5) und/oder einen zentralen Bewegungssensor, in Richtung einer Objektachse der geometrischen Form gesehen, insbesondere in Richtung einer Zylinderachse gesehen, zentral an dem Objektbereich angeordnet ist.

7. Verfahren gemäß Anspruch 6,

wobei die aktuelle Position des Objektbereichs bestimmt wird, indem ein Kreis, der durch das Zentrum des Objektbereichs verläuft und einem Querschnitt der geometrischen Form, insbesondere des Zylinders, entspricht, konstruiert wird,
wobei der Kreis mit Hilfe von Sensordaten des zumindest einen zentralen Sensors und mit Sensordaten von zumindest zwei weiteren Sensoren, insbesondere von zwei weiteren Magnetfeldsensoren (5) und/oder Bewegungssensoren, die auf die Ebene des Kreises projiziert werden, konstruiert wird.

8. Verfahren gemäß Anspruch 7,
wobei eine Position des Objektbereichs über den Kreismittelpunkt bestimmt wird.

9. Verfahren gemäß Anspruch 7 oder 8,
wobei eine Größe des Objektbereichs über einen Radius des Kreises bestimmt wird.

10. Verfahren gemäß einem der Ansprüche 7 bis 9,
wobei eine Orientierung des Objektbereichs anhand einer Achse des zumindest einen zentralen Sensors bestimmt wird, die parallel zu einer Objektachse, insbesondere Zylinderachse, verläuft.

11. Messvorrichtung umfassend

eine Trägereinheit (1), insbesondere Magnetresonanztomographie-Körperspule, und zumindest einen Bewegungssensor, insbesondere Beschleunigungssensor (3), bevorzugt zumindest drei Bewegungssensoren,
wobei der zumindest eine Bewegungssensor an der Trägereinheit (1) befestigt ist,
wobei die Trägereinheit (1) im Wesentlichen zylinderförmig ist oder in eine Zylinderform formbar ist.

12. Messvorrichtung gemäß Anspruch 11,
wobei die Messvorrichtung einen oder mehrere Magnetfeldsensoren (5) umfasst, die an der Trägereinheit (1) befestigt sind.

13. Messvorrichtung gemäß einem der Ansprüche 11 bis 12, wobei die Zylinderform der Trägereinheit (1) oder die Zylinderform in die die Trägereinheit (1) formbar ist eine Zylinderachse aufweist, wobei zumindest einer der Sensoren, insbesondere ein Magnetfeldsensor (5) und/oder Bewegungssensor, in Richtung der Zylinderachse gesehen im Wesentlichen zentral auf der Trägereinheit (1) angeordnet ist.

14. Messvorrichtung gemäß einem der Ansprüche 11 bis 13, wobei die Trägereinheit (1) eine Markierung aufweist, die zumindest eine definierte, insbesondere die zentrale, Position auf der Zylinderachse markiert.

15. Magnetresonanztomographiesystem umfassend eine Messvorrichtung gemäß einem der Ansprüche 11 bis 14 und einen Untersuchungstunnel (6),
wobei das Magnetresonanztomographiesystem dazu konfiguriert ist, mit dem zumindest einen Bewegungssensor die Bewegung eines Objektbereichs in dem Untersuchungstunnel (6) zu verfolgen, insbesondere gemäß einem Verfahren nach einem der Ansprüche 1 bis 10.

FIG 1

101    102    103    104

FIG 2

211

212

213

214

215

216

FIG 3

FIG 4

FIG 5

|B0|

FIG 6

FIG 7

# EP 4 571 344 A1

| | Europäisches Patentamt<br>European Patent Office<br>Office européen des brevets | **EUROPÄISCHER RECHERCHENBERICHT** | **Nummer der Anmeldung**<br>**EP 23 21 6088** |
|---|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 3 349 030 A1 (SAMSUNG ELECTRONICS CO LTD [KR]) 18. Juli 2018 (2018-07-18)<br>* Abbildungen 8-14 *<br>* Absatz [0087] – Absatz [0233] *<br>----- | 1,5-15 | INV.<br>G01R33/565 |
| X,D | DE 10 2016 203255 A1 (SIEMENS HEALTHCARE GMBH [DE]) 31. August 2017 (2017-08-31)<br>* das ganze Dokument *<br>----- | 1,3-10 | |
| X | EP 3 328 275 A1 (UNIV CAPE TOWN [ZA]) 6. Juni 2018 (2018-06-06)<br>* das ganze Dokument *<br>----- | 1-10 | |
| A | DE 10 2021 202118 A1 (SIEMENS HEALTHCARE GMBH [DE]) 8. September 2022 (2022-09-08)<br>* das ganze Dokument *<br>----- | 1-15 | |
| | | | **RECHERCHIERTE SACHGEBIETE (IPC)**<br><br>G01R<br>A61B |

Der vorliegende Recherchenbericht wurde für alle Patentansprüche erstellt

| Recherchenort | Abschlußdatum der Recherche | Prüfer |
|---|---|---|
| München | 2. Mai 2024 | Durst, Markus |

## ANHANG ZUM EUROPÄISCHEN RECHERCHENBERICHT
## ÜBER DIE EUROPÄISCHE PATENTANMELDUNG NR.

EP 23 21 6088

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-05-2024

| Im Recherchenbericht angeführtes Patentdokument | Datum der Veröffentlichung | Mitglied(er) der Patentfamilie | Datum der Veröffentlichung |
|---|---|---|---|
| EP 3349030 A1 | 18-07-2018 | CN 108181596 A | 19-06-2018 |
| | | EP 3349030 A1 | 18-07-2018 |
| | | KR 20180065455 A | 18-06-2018 |
| | | US 2018164392 A1 | 14-06-2018 |
| DE 102016203255 A1 | 31-08-2017 | DE 102016203255 A1 | 31-08-2017 |
| | | US 2017248665 A1 | 31-08-2017 |
| EP 3328275 A1 | 06-06-2018 | CN 107920774 A | 17-04-2018 |
| | | EP 3328275 A1 | 06-06-2018 |
| | | JP 2018520815 A | 02-08-2018 |
| | | US 2018184940 A1 | 05-07-2018 |
| | | WO 2017021804 A1 | 09-02-2017 |
| | | ZA 201708020 B | 26-06-2019 |
| DE 102021202118 A1 | 08-09-2022 | CN 115024711 A | 09-09-2022 |
| | | DE 102021202118 A1 | 08-09-2022 |
| | | US 2022280117 A1 | 08-09-2022 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016203255 A1
- US 20170248665 A1

- EP 4170375 A1